# EUROPEAN PATENT APPLICATION

(11) **EP 1 955 680 A1**
(43) Date of publication of application: **13.08.2008**
(21) Application number: 06832621.4
(22) Date of filing: 14.11.2006
(51) Int. Cl.: A61F 2/84, A61F 2/06

(54) **INSTRUMENT FOR DILATING BLOOD CHANNEL AND INSTRUMENT FOR TREATING AORTIC DISSECTION**

(30) Priority: 14.11.2005 JP 2005329325; 14.11.2005 JP 2005329328
(71) Applicant: JMS Co., Ltd., Hiroshima-shi, Hiroshima 730-8652 (JP)
(72) Inventor: ORIHASHI, Kazumasa, Hiroshima-shi, Hiroshima 734-0002 (JP); HAYASHI, Shuro, Hiroshima-shi, Hiroshima 730-8652 (JP)
(74) Representative: Collin, Jérôme
(86) International application number: PCT/JP2006/322665
(87) International publication number: WO 2007/055382

(57) **Abstract**

A bloodstream dilating device 1 is composed of an elastic member 2 extending in a direction of blood flow in a true lumen 115. The elastic member 2 pushes the inner wall of the true lumen 15 outward to dilate the bloodstream. An aortic dissection treating device includes an obturator and a fixing member for fixing the obturator to the inner layer of an aorta. The obturator is inserted up to the point that it can cover an entry port formed in the aorta. The obturator is deformed along the periphery of the entry port in the inner layer by blood pressure to occlude the entry port.

## Description

### Technical Field

As one of aortic diseases caused due to arterial sclerosis and the like, occurrence of aortic dissection is increasing recently. The aortic dissection is a pathologic condition that the blood flowing in an aorta from an entry port formed due to breakage of a part of the inner layer of the aorta flows between the inner layer and the outer layer thereof to form a bloodstream as a false lumen between the inner layer and the outer layer besides a true lumen forming a bloodstream in which the blood flows in the normal condition.

In the aortic dissection, the blood may flow into the false lumen more than the true lumen according to the size of the entry port or the progress of arterial sclerosis. In this state, the blood flowing in the false lumen pushes the inner layer between the true lumen and the false lumen toward the true lumen. This expands the outer layer to expand the bloodstream of the false lumen while narrowing the bloodstream of the true lumen. When the bloodstream of the true lumen is narrowed in the vicinity of the visceral arteries branching from the aorta, the amount of the blood flowing into the visceral arteries reduces to make the viscera to fall in an ischemic state even though the visceral arteries do not occlude. A significant symptom appearing when the viscera are in such a ischemic state is non occlusive mesenteric ischemia which necessitates incision of a large volume of the intestine because of ischemia of the intestinal tissue.

In order to suppress the ischemia state of the viscera caused due to the aortic dissection, a procedure may be taken in which the inner layer between the true lumen and the false lumen on the peripheral side of the entry port is incised with the use of a cutting tool for the inner layers of the arteries as disclosed in Patent Document 1, for example, to make the bloodstream of the true lumen and the bloodstream of the false lumen to communicate with each other for allowing the blood flowing in the false lumen to flow into the bloodstream of the true lumen.

The aforementioned pathologic condition of the aortic dissection is classified into: DeBakey I type aortic dissection in which the false lumen ranges from the ascending aorta to the thoracic descending aorta; DeBakey II type aortic dissection in which the false lumen remains in the ascending aorta; DeBakey IIIa type aortic dissection in which the false lumen remains on the peripheral side in the blood flow direction of the left subclavian artery in the thoracic descending aorta; and DeBakey IIIb type in which the false lumen ranges from the thoracic descending aorta to the ventral aorta.

Of the pathologic conditions of the aortic dissection, DeBakey I type and DeBakey II type aortic dissections, which are also called Stanford A type, in which the false lumen ranges to the ascending aorta causes the false lumen to press the inlet of a coronary artery or to bend the aortic valve to make the patient to fall in a lethal state in an acute phase, and accordingly, emergency surgical treatment is necessitated. In contrast, in DeBakey IIIa type and DeBakey IIIb type aortic dissections, which are also called Stanford B type, in which no false lumen is formed in the ascending aorta, a treatment course of hypotensive therapy in which a patient is kept in a strictly resting state with his/her blood pressure lowered is taken in general in the medical sites unless none of aneurysm rupture, bosslation, perfusion disorder to any branching artery, and the like occurs. In the hypotensive therapy, strict management under an ICU for about one week is necessary from the development to the time when the blood in the false lumen forms a thrombus to allow the dissection symptom to become stable. The management under the ICU for about one week may cause a complication to a patient, such as a disorder state, atelectasis, pneumonia, or the like. If such a complication is caused, the management under the ICU lasts long to prolong the hospitalization for three to four weeks.

To tackle this problem, in treating the DeBakey IIIa type or DeBakey IIIb type aortic dissection under the hypotensive therapy, an artificial vessel of intravascular indwelling type as disclosed in, for example, Patent Document 2, that is, a Stent graft may be used as an entry port obturator to be inserted in the true lumen of an aorta. When the entry port is occluded by inserting the obturator up to a part of the true lumen corresponding to the entry port, the flow of the blood flowing into the false lumen from the true lumen through the entry port is blocked. As a result, less blood remains in the false lumen to form a thrombus early, so that the dissection symptom becomes stable in an early phase. Accordingly, the duration of the management under the ICU can be shortened to shorten the hospitalization.
Patent Document 1: Japanese Unexamined Patent Application Publication 5-220158
Patent Document 2: Japanese Unexamined Patent Application Publication 2005-58434

### Disclosure of the Invention

### Problems that the Invention is to Solve

However, even when the bloodstream of the false lumen is allowed to communicate with the bloodstream of the true lumen with the use of the cutting tool in Patent Document 1, it is difficult to increase the amount of the blood flowing into the bloodstream of the true lumen from the bloodstream of the false lumen because the inner layer between the true lumen and the false lumen has been pushed toward the true lumen by the blood flowing in the bloodstream of the false lumen through the entry port and the bloodstream of the true lumen has been already narrowed. As a result, the bloodstream of the true lumen remains narrowed to disable sufficient suppression of ischemia of the viscera.

Further, even in the aortic dissections classified in DeBakey IIIa type and DeBakey IIIb type, the progress of the arterial sclerosis, the size and the location of the entry port, and the shape of the false lumen are different from one patient to another, and accordingly, the shape of a part of the true lumen where the obturator is to be inserted is different among patients largely. Therefore, for carrying out the hypotensive therapy for the aortic dissections with the use of the obturator as in Patent Document 2, it is necessary for securely occluding the entry port by the obturator to design the shape of the obturator precisely for each patient so that the shape thereof agrees with the shape of the true lumen. In other words, the hypotensive therapy using the obturator in Patent Document 2 can be carried out only in a limited medical site equipping a device, designing equipment, and the like capable of precisely grasping the shape of the true lumen.

In view of the fact that ischemia of the viscera cannot be suppressed by a treatment using the tool in Patent Document 1, the present invention has its principal object of suppressing ischemia of viscera in such a manner that the amount of the blood flowing in a bloodstream of the true lumen in an aorta in aortic dissection is increased by expanding the bloodstream of the true lumen from the inside thereof.

In view of the fact that the hypotensive therapy using the obturator in Patent Document 2 can be carried out only in the limited medical site, the second object of the present invention is to enable to the hypotensive therapy be carried out in various medical sites by enabling entry port occlusion without necessitating precise design of an entry port obturator used for the hypotensive therapy for aortic dissection for each patient.

### Means for Solving the Problems

In order to attain the principal object, in the present invention, an elastic member is used for pushing the inner wall of the true lumen outward of the true lumen.

Specifically, the first aspect of the present invention is directed to a bloodstream dilating device to be indwelled in a bloodstream of a true lumen of an aorta where aortic dissection, which forms a false lumen in the aorta beside the true lumen by blood in the aorta flowing between an inner layer and an outer layer of the aorta, occurs.

The bloodstream dilating device includes an elastic member for pushing an inner wall of the true lumen outward of the true lumen.

With the above arrangement, the elastic member pushes the inner wall of the true lumen outward to dilate the bloodstream of the true lumen, thereby increasing the blood amount in the bloodstream thereof. Further, pushing of the inner layer between the true lumen and the false lumen toward the false lumen narrows the bloodstream of the false lumen to decrease the amount of the blood flowing into the bloodstream of the false lumen through the entry port while the decreased amount of the blood flows into the bloodstream of the true lumen. This increases the amount of the blood flowing in the bloodstream of the true lumen to secure the amount of the blood flowing to the visceral arteries.

Referring to a second aspect of the present invention, in the first aspect, the elastic member includes a plurality of elastic members, and the elastic members are formed so as to extend linearly in a direction of a blood flow in the true lumen and are spaced in a peripheral direction of the true lumen.

In the above arrangement, each elastic member extends in the blood flow direction of the true lumen, so that the elastic members less inhibit the blood flow in the bloodstream of the true lumen. A plurality of parts of the peripheral wall of the true lumen are pushed by the elastic members.

Referring to a third aspect of the present invention, in the second aspect, one end parts of the elastic members are combined with each other while the other end parts thereof are combined with each other, and intermediate parts of the elastic members are formed so as to push the inner wall of the true lumen.

In the above arrangement, the elastic members are integrated at the respective end parts thereof to be prevented from separating from each other in an operation for indwelling the bloodstream dilating device into the bloodstream of the true lumen.

Referring to a fourth aspect of the present invention, in the second invention, one end parts of the elastic members are combined with each other while the other end parts thereof are formed so as to push the inner wall of the true lumen.

In the above arrangement, the elastic members are combined and integrated at the one end parts thereof to be prevented from separating from each other in an operation for indwelling the bloodstream dilating device into the bloodstream of the true lumen. Further, the other end parts of the elastic members are formed so as to be positioned around the outer periphery of the bloodstream for pushing the inner wall of the true lumen. Hence, the elastic members less inhibit the blood flow in the bloodstream of the true lumen.

Referring to a fifth aspect of the present invention, in the first aspect, the elastic member is provided with a protruding portion formed so as to be caught at the inner wall of the true lumen.

In the above arrangement, the protruding portion of the elastic member is caught at the inner wall of the true lumen, so that displacement of the elastic member toward the peripheral side in the bloodstream by the blood flow can be suppressed.

Referring to a sixth aspect of the present invention, in the fifth aspect, the protruding portion points toward the peripheral side in a direction of a blood flow in the true lumen.

With the above arrangement, the elastic member is pushed by the blood flow in the bloodstream in the direction that the protruding portion points, so that the protruding portion hardly separates from the inner wall of the true lumen.

In order to attain the second object, in the present invention, a film-shaped obturator is fixed to the inner layer of an aorta to occlude an entry port by allowing the obturator to be in contact with the periphery of the entry port by the pressure of the blood.

Specifically, a seventh aspect of the present invention is directed to an aortic dissection treating device for treating aortic dissection occurring due to flow of blood in an aorta flowing between an inner layer and an outer layer of the aorta through an entry port formed in the inner layer of the aorta, the aorta dissection treating device.

The aortic dissection treating device includes: an obturator in a film shape larger than the entry port for occluding the entry port by being inserted into the true lumen of the aorta and being in contact with a periphery of the entry port in the inner layer by pressure of the blood flowing in the aorta; and a fixing member for fixing the obturator to the inner layer.

With the above arrangement, the fixing member can fix the obturator to the inner layer of the aorta when the obturator is inserted to the point of the true lumen of the aorta corresponding to the entry port. The obturator fixed to the inner layer is in the film shape to be readily deformed along the shape of and be in contact with the periphery of the entry port of the inner layer by the pressure of the blood flowing in the true lumen. Even though the shape of the true lumen is different from one patient to another, the obturator can occlude the entry port since the pressure of the blood allows the obturator to be in contact with the periphery of the entry port in the inner layer, thereby eliminating the need for precise design of the obturator. The obturator blocks the flow of the blood flowing into the false lumen from the true lumen through the entry port, with a result that the blood in the false lumen forms a thrombus in an early phase to stabilize the condition of the dissection state.

Referring to an eighth aspect of the present invention, in the seventh aspect, the fixing member is composed of an elastic member formed so as to be elastically deformed radially outward of the aorta to push the inner layer, and the obturator is mounted on a part of the elastic member which pushes the inner layer.

With the above arrangement, the elastic member pushes the inner layer to allow the obturator to be held between the elastic member and the inner layer.

Referring to a ninth aspect of the present invention, in the eighth aspect, the elastic member is formed so as to extend in a direction of the flow of the blood in the aorta.

In the above arrangement, the elastic member extends in the blood flow direction to inhibit less the blood flow of the true lumen.

Referring to a tenth aspect of the present invention, in the ninth aspect, the elastic member includes a plurality of elastic members spaced in a peripheral direction of the true lumen.

With the above arrangement, the plural parts of the obturator are held between the elastic member and the inner layer.

Referring to an eleventh aspect of the present invention, in the seventh aspect, the elastic member is provided with an engaging part to be engaged with a withdrawing tool.

With the above arrangement, when the withdrawing tool is engaged with the engaging part for withdrawing the aortic dissection treating device from the true lumen after the treatment using the aortic dissection treating device is finished, unfailing and easy withdrawal by the withdrawing tool is attained.

Referring to a twelfth aspect of the present invention, in the seventh aspect, the obturator is in a cylindrical shape.

With the above arrangement, the cylindrical obturator extends along the periphery of the true lumen. Accordingly, when the obturator is inserted up to the point of the true lumen corresponding to the entry port, the entry port can be covered without failure with no rotation of the obturator in the peripheral direction of the true lumen in the true lumen necessitated.

Referring to a thirteenth aspect of the present invention, in the seventh aspect, the obturator is cut across a peripheral direction of the true lumen.

In the case where the entry port is formed near a branching artery of an aorta, when the obturator with the above arrangement is inserted up to the point of the true lumen corresponding to the entry port and the cut end of the obturator is allowed to agree with the inlet of the branching artery, the inlet of the branching artery is prevented from being occluded by the obturator.

Referring to a fourteenth aspect of the present invention, in the seventh aspect, the obturator is provided with a reinforcing part extending in a direction of the flow of the blood.

With the above arrangement, the reinforcing part reinforces the obturator to thus suppress a state that the obturator turns into the entry port by the flow of the blood flowing into the false lumen through the entry port.

Referring to a fifteenth aspect of the present invention, in the seventh aspect, the obturator is made of a bioabsorbable material.

With the above arrangement, after the entry port is occluded by allowing the obturator to be in contact with the periphery of the entry port, the obturator is gradually absorbed into the inner layer and disappears from the inside of the aorta in due course.

### Effects of the Invention

In the first aspect of the present invention, the elastic member pushes the inner wall of the true lumen outward to dilate the bloodstream of the true lumen while narrowing the bloodstream of the false lumen. This increases the amount of the blood flowing in the bloodstream of the true lumen to secure the amount of the blood flowing to the visceral arteries, thereby suppressing ischemia of the viscera.

In the second aspect of the present invention, each elastic member extends in the blood flow direction of the true lumen, so that the elastic members less inhibit the blood flow in the bloodstream of the true lumen. A plurality of parts of the peripheral wall of the true lumen are pushed by the elastic members, with a result that the bloodstream is dilated definitely.

In the third aspect of the present invention, the elastic members are combined and integrated at the one end parts thereof with each other and at the other end parts thereof with each other to facilitate an operation for indwelling the bloodstream dilating device into the bloodstream of the true lumen.

In the fourth aspect of the present invention, the elastic members are integrated at the one end parts thereof to facilitate an operation for indwelling the bloodstream dilating device into the bloodstream of the true lumen. Further, the other end parts of the elastic members are positioned around the periphery of the bloodstream, and hence, the elastic members inhibit further less the blood flow in the bloodstream of the true lumen.

According to the fifth aspect of the present invention, the protruding portion of the elastic member is caught at the inner wall of the true lumen, so that displacement of the bloodstream dilating device toward the peripheral side of the bloodstream can be suppressed. This attains desired dilation of the bloodstream of the true lumen.

In the sixth aspect of the present invention, the protruding portion points toward the peripheral side of the blood flow direction to suppress falling off of the protruding portion from the inner wall of the true lumen.

In the seventh aspect of the present invention, the fixing member fixes to the inner layer the film-shaped obturator inserted in the true lumen of the aorta so as to allow the obturator to be in contact with the periphery of the entry port in the inner layer by the pressure of the blood in the true lumen. Accordingly, the obturator can occlude the entry port without necessitating precise design of the obturator even though the shape of the true lumen is different from one patient to another. This eliminates the need to prepare a device, equipment, and the like for precisely designing the obturator, thereby enabling the hypotensive therapy to be carried out in various medical sites.

In the eighth aspect of the present invention, the elastic member for pushing the inner layer is composed of the fixing member and the obturator is mounted on the inner layer side of the elastic member, thereby suppressing displacement of the obturator to enable definite occlusion of the entry port.

In the ninth aspect of the present invention, the elastic member extends in the blood flow direction to inhibit less the blood flow in the true lumen, thereby securing the amount of the blood in the true lumen.

In the tenth aspect of the present invention, the plural parts of the obturator can be held between the elastic members and the inner layer to allow the obturator to occlude the entry port definitely.

In the eleventh aspect of the present invention, engagement of the withdrawing tool with the engaging part leads to unfailing and easy withdrawal of the aortic dissection treating device from the true lumen.

In the twelfth aspect of the present invention, the obturator in a peripherally extending and cylindrical shape can occlude the entry port without necessitating rotation of the obturator in the peripheral direction of the true lumen within the true lumen. This facilitates a treatment for occluding the entry port by the obturator.

In the thirteenth aspect of the present invention, the obturator is cut across the peripheral direction of the true lumen. Accordingly, when the cut end of the obturator is allowed to agree with the inlet of a branching artery, the blood flow to the branching artery can be secured with the entry port occluded.

According to the fourteenth aspect of the present invention, turning of the obturator into the entry port can be suppressed to lead to definite occlusion of the entry port.

In the fifteenth aspect of the present invention, the obturator is made of a bioabsorbable material. Accordingly, after the entry port is occluded, the obturator disappears in the aorta as time passes. This eliminates the need to carry out a treatment for taking out the obturator from the aorta to thus attain low invasion treatment.

### Brief Description of the Drawings

[FIG. **1]** FIG. **1** shows a bloodstream dilating device in accordance with Embodiment 1 of the present invention, wherein FIG. **1(a)** is a perspective view and FIG. **1(b)** is a plan view as viewed from the other end part in the center line direction of the blood flow dilating device.
[FIG. 2] FIG. 2 is a schematic view showing an aortic lines where aortic dissection occurs.
[FIG. 3] FIG. 3 is a perspective view of a ventral aorta where aortic dissection occurs.
[FIG. 4] FIG. 4 is a vertical sectional view of the ventral aorta where aortic dissection occurs.
[FIG. 5] FIG. 5 is a view for explaining a state where the bloodstream dilating device is accommodated in a catheter.
[FIG. 6] FIG. 6 is a view corresponding to FIG. 4 which shows a state where the bloodstream dilating device accommodated in the catheter is inserted into the ventral aorta.
[FIG. **7]** FIG. **7** is a view corresponding to FIG. **4** which shows a state where a part of the bloodstream dilating device is pushed out from the catheter.
[FIG. **8]** FIG. **8** is a view corresponding to FIG. **4** which shows a state where the bloodstream dilating device dilates the bloodstream in a true lumen.
[FIG. **9**] FIG. **9** is a view corresponding to FIG. **1(a)** in accordance with Modified Example 1 of Embodiment 1.
[FIG. **10]** FIG. **10** is a view corresponding to FIG. **1(a)** in accordance with Modified Example 2 of Embodiment 1.
[FIG. **11]** FIG. **11** is a view corresponding to FIG. **8** in accordance with Modified Example 2 of Embodiment 1.
[FIG. **12]** FIG. **12** is a view corresponding to FIG. **8** which shows a state where a bloodstream dilating device in accordance with Modified Example 2 of Embodiment 1 is being accommodated in a withdrawing catheter.
[FIG. **13]** FIG. **13** is a view corresponding to FIG. **8** which shows a state where the bloodstream dilating device in accordance with Modified Example 2 of Embodiment 1 is accommodated in the withdrawing catheter.
[FIG. **14**] FIG. 14 is a view corresponding to FIG. **1(a)** in accordance with Modified Example 3 of Embodiment 1.
[FIG. **15]** FIG. **15** is a view corresponding to FIG. **8** in accordance with Modified Example 3 of Embodiment 1.
[FIG. **16]** FIG. **16** is a perspective view of an aortic dissection treating device in accordance with Embodiment 2 of the present invention.
[FIG. **17**] FIG. **17(a)** is a perspective view of a fixing member, and FIG. **17(b)** is a perspective view of an obturator.
[FIG. **18]** FIG. **18** is a schematic view showing an aortic lines where aortic dissection occurs.
[FIG. **19]** FIG. **19** is a view for explaining a state where the aortic dissection treating device is accommodated in a catheter.
[FIG. **20]** FIG. **20** is an enlarged view of FIG. **8** which shows a state where a catheter accommodating the aortic dissection treating device is inserted into a true lumen.
[FIG. **21]** FIG. **21** is a view corresponding to FIG. **20** which shows a state where a part of the aortic dissection treating device is pushed out from the catheter.
[FIG. **22]** FIG. **22** is a view corresponding to FIG. **20** which shows a state where the aortic dissection treating device is indwelled in the true lumen.
[FIG. **23]** FIG. **23** is a view corresponding to FIG. **20** which shows a state where the aortic dissection treating device is being withdrawn by a withdrawing tool.
[FIG. **24]** FIG. **24** is a view corresponding to FIG. 16 in accordance with Modified Example 1 of Embodiment 2.
[FIG. **25**] FIG. **25** is a view corresponding to FIG. **17(a)** in accordance with Modified Example 2 of Embodiment 2.
[FIG. **26]** FIG. **26** is a view corresponding to FIG. **16** in accordance with Modified Example 3 of Embodiment 2.
[FIG. **27]** FIG. **27** is a view corresponding to FIG. **17(a)** in accordance with Modified Example 4 of Embodiment 2.

### Explanation of Reference Numerals

- **1**: bloodstream dilating device
- **2**: elastic member
- **3**: one-side curved part
- **4**: other-side curved part
- **5**: straight part
- **6**: protruding portion
- **60**: aortic dissection treating device
- **61**: obturator
- **62**: fixing member
- **63**: striped protrusion (reinforcing part)
- **64**: elastic member
- **67**: hook (engaging part)
- **100**: aorta
- **114**: false lumen
- **115**: true lumen

### Best Mode for Carrying out the Invention

Embodiments of the present invention will be described below in detail with reference to the accompanying drawings. It is noted that the following preferred embodiments describe mere substantial examples and are not intended to limit the present invention, applicable objects, and use thereof.

### (Embodiment 1)

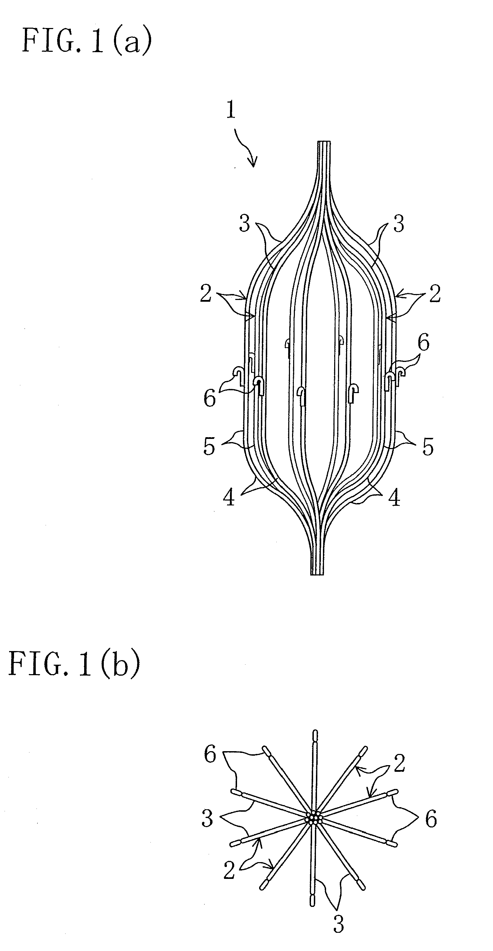

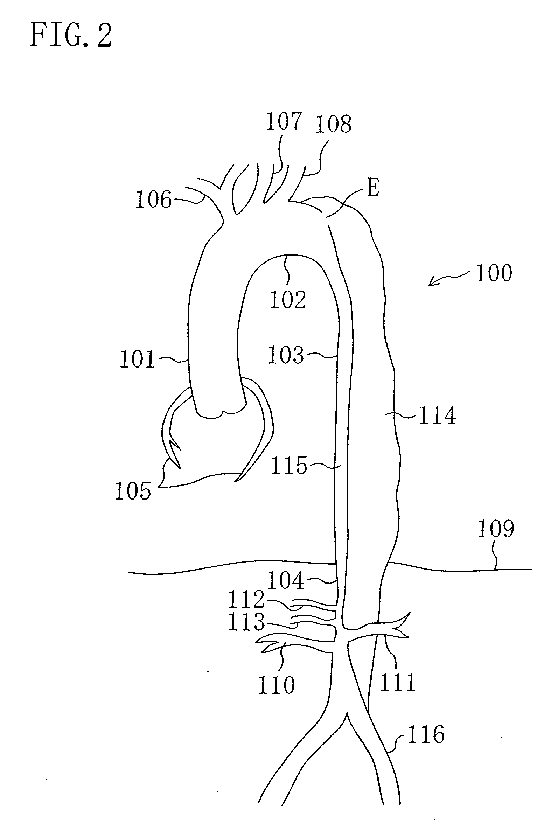

FIG. **1** shows a bloodstream dilating device **1** in accordance with Embodiment 1 of the present invention. The bloodstream dilating device **1** is to be indwelled in a true lumen 115 (shown in FIG. **2** to FIG. **4****)** of an aorta **100** where aortic dissection occurs for dilating a bloodstream in the true lumen **115.**

The bloodstream dilating device **1** is in a cylindrical basket shape of a combination of a plurality of linear elastic members **2.** The bloodstream dilating device 1 has a length in the center line direction (the vertical direction in FIG. **1(a)****)** of approximately 40 mm to approximately 60 mm. In Embodiment 1, the number of the elastic members 2 is set at ten. Wherein, the number of the elastic members 2 may be set at eight or nine, for example.

The elastic members **2** are arranged so that the longitudinal direction thereof substantially agrees with the blood flow direction of the true lumen **115** and so as to be spaced in the peripheral direction of the true lumen **115** when the bloodstream dilating device **1** is indwelled in the bloodstream of the true lumen **115.** The material of the elastic members **2** may be a metal material or a resin material having a spring characteristic, such as a Ni-Ti alloy, a stainless steel, or the like, for example.

One end parts of the elastic members **2** (the lower end part of the bloodstream dilating device shown in FIG. **1(a)****)** are located at one end part on or near the center line of the bloodstream dilating device **1.** The one end parts of the elastic members **2** are combined by a joining method, such as welding. As well, the other end parts of the elastic members **2** (the upper end part of the bloodstream dilating device shown in FIG. **1(a)****)** are located at the other end part on or near the center line of the bloodstream dilating device **1.** The other end parts of the elastic members **2** are combined also.

Each part of the elastic members **2** opposite the corresponding other end part thereof forms an other-side curved part **3** curved so as to separate radially from the center line of the bloodstream dilating device **1.** As well, each part of the elastic members **2** opposite the corresponding one end part forms a one-side curved part **4** curved so as to separate radially from the center line of the bloodstream dilating device **1.** Accordingly, the outer diameter of the one side in the center line direction of the bloodstream dilating device **1** expands as it goes toward the other side while the outer diameter of the other side in the center line direction thereof expands as it goes toward the one side thereof.

Between the other-side curved parts **3** and the one-side curbed parts **4,** there are formed straight parts **5** extending substantially straight in parallel with the center line of the bloodstream dilating device **1.** The straight parts **5** are to push the inner wall of the true lumen **115** outward. The diameter of a part of the bloodstream dilating device **1** corresponding to the straight part **5** is set at approximately 20 mm to approximately 30 mm, which is shorter than the length in the center line direction of the bloodstream dilating device **1.**

Each elastic member **2** is provided with a protruding portion **6** formed so as to be caught at the inner wall of the true lumen **115.** Each protruding portion **6** extends toward the other side in the center line direction of the bloodstream dilating device **1** along the corresponding straight part 5, curves radially outward of the bloodstream dilating device **1,** and extends then toward the one side in the center line direction thereof. The parts of the protruding portions **6** which extend along the straight parts **5** are fixed to the elastic members **2.**

Description will be given next on the case for dilating the true lumen 115 of the aorta where the aortic dissection occurs with the use of the thus constructed bloodstream dilating device **1.** First, an aorta **100** and a pathologic condition of the aortic dissection will be described with reference to FIG. **2** to FIG. **4****.** The aorta **100** includes an ascending aorta **101,** an arcuate aorta **102,** a thoracic descending aorta **103,** and a ventral aorta **104.** Coronary arteries **105** branch from the starting part of the ascending aorta **101.** A right brachiocephalic artery **106,** a left common carotid artery **107,** and a left subclavian artery **108** branch from the arcuate aorta **102.** Four main ventral branches of a left renal artery **110,** a right renal artery **111,** a celiac artery **112,** and a superior mesenteric artery **113** branch from the ventral aorta **104.** Reference numeral **109** denotes a diaphragm.

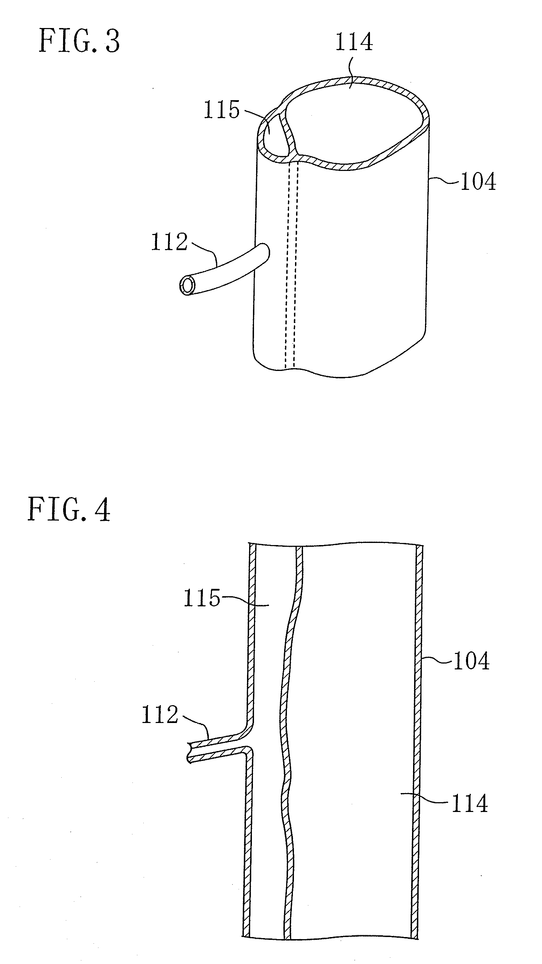

When an entry port **E** is formed in the inner layer of the arcuate aorta **102** on the peripheral side of the branch part of the left subclavian artery **108,** the blood in the arcuate aorta **102** flows between the inner layer and the outer layer through the entry port **E.** The blood flowing between the inner layer and the outer layer breaks the inner layer to peel the inner layer from the outer layer toward the peripheral side, thereby forming a false lumen **114,** as shown in FIG. **3** and FIG. **4****.** In other words, the aortic dissection forms the false lumen **114** beside the true lumen **115** forming a normal bloodstream in the arcuate aorta **102,** the thoracic descending aorta **103,** and the ventral aorta **104.** The inner diameter of the true lumen **115** is smaller than that of the normal arcuate aorta **104** and that of the false lumen **114** and is especially narrowed in the vicinity of the celiac artery **112** and the superior mesenteric artery **113.** The entry port **E** is in a slit shape long in the peripheral direction of the arcuate aorta **102** and has a dimension in the longitudinal direction of approximately 20 mm to approximately 30 mm and a dimension in the widthwise direction of approximately 10 mm.

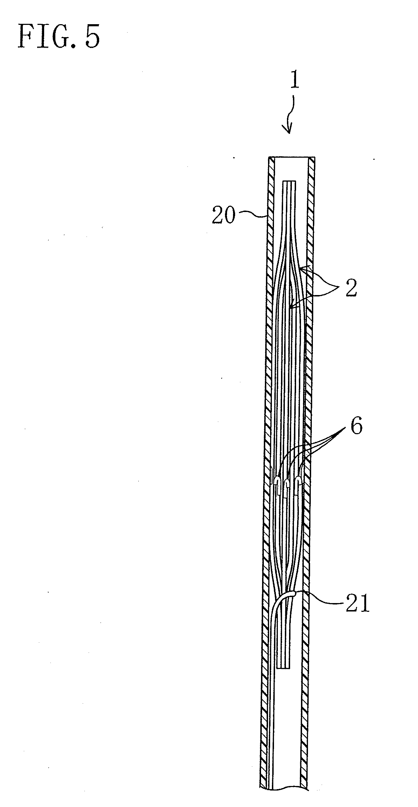

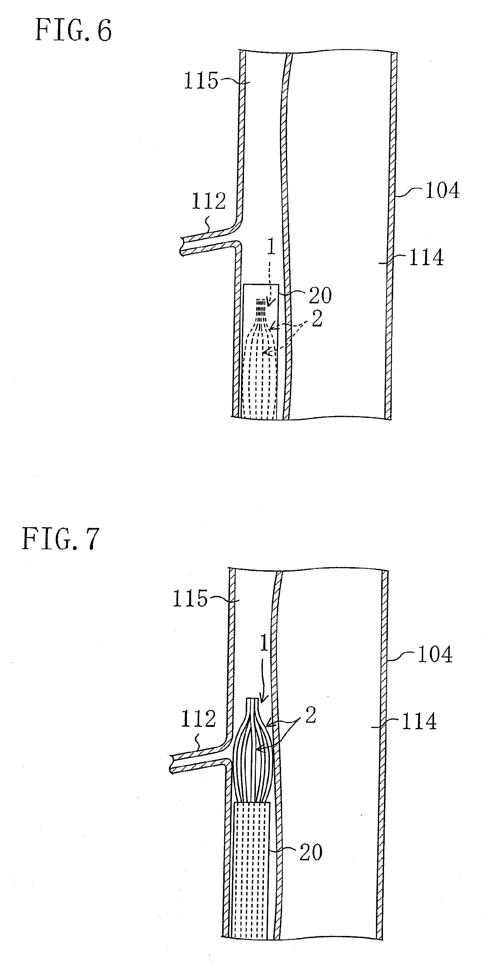

The bloodstream dilating device **1** is inserted up to the ventral aorta **104** with it accommodated in a catheter **20,** as shown in FIG. **5** and FIG. **6****.** The catheter **20** is one used in the medical fields and is made of a flexible resin material. For inserting the bloodstream dilating device **1** into the catheter **20,** the other-side curved part **3** and the one-side curved part **4** of each elastic member **2** are elastically deformed radially inward to reduce the outer diameter of the bloodstream dilating device **1.** Then, the bloodstream dilating device **1** is inserted from one end in the center line direction thereof into a distal end part in the insertion direction of the catheter **20** to be accommodated in the catheter **20.** The distal ends of the protruding portions **6** point toward the base end of the catheter **20** when the bloodstream dilating device **1** is accommodated in the catheter **20.**

In the catheter **20,** a wire **21** is inserted of which one end is engaged with the one end part of the bloodstream dilating device 1 and of which other end is protruded out from the base end of the catheter **20.**

A skin of a patient's lower limb (not shown) is incised, and the catheter **20** is inserted into an artery of the lower limb. As shown in FIG. **6****,** the catheter **20** is moved in the insertion direction in the artery to insert the distal end part thereof from the common iliac artery **116** (shown in FIG. **2****)** to the ventral aorta **104.** Then, as shown in FIG. **7****,** the other end part of the wire **21** is pushed to the catheter **20** so that the bloodstream dilating device **1** is pushed out from the distal end of the catheter **20** into the true lumen **115.**

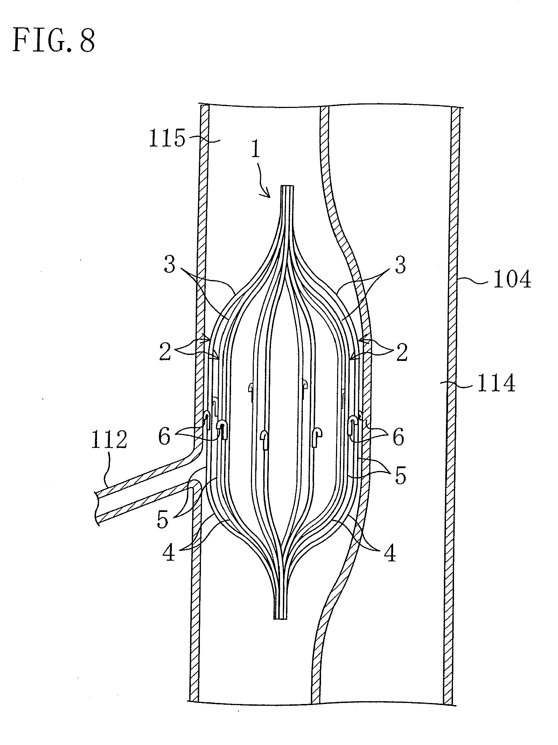

A part of the bloodstream dilating device **1** which is pushed out from the catheter 20 is deformed at the elastic members **2** thereof radially outward. When the bloodstream dilating device **1** is pushed out completely from the catheter **20,** as shown in FIG. **8****,** the elastic members **2** recovers to the original shapes before accommodation thereof in the catheter **20,** so that the straight part **5** of each elastic member pushes the inner wall of the true lumen **115** outward. This dilates the bloodstream of the true lumen **115** to increase the amount of the blood flowing in the bloodstream thereof.

In this state, the inner layer between the true lumen **115** and the false lumen **114** is pushed toward the false lumen **115** to narrow the bloodstream of the false lumen **114.** This decreases the amount of the blood flowing in the bloodstream of the false lumen **114,** with a result that the decreased amount of the blood flows into the true lumen **115.** The elastic members **2** are in a linear shape to inhibit less the blood flow in the bloodstream of the true lumen **115.** Accordingly, the amount of the blood flowing in the bloodstream of the true lumen **115** increases to secure the amount of the blood flowing to the visceral arteries, such as the celiac artery **112,** the superior mesenteric artery **113,** and the like.

Further, the protruding portions **6** of the elastic members **2** are caught at the inner wall of the true lumen **115.** Since the bloodstream dilating device 1 is inserted so that the distal ends of the protruding portions **6** point toward the peripheral side of the true lumen **115,** the direction in which the blood flow in the bloodstream pushes the elastic members **2** agrees with the direction in which the distal ends of the protruding portions **6** point. Accordingly, the protruding portions **6** bite the inner wall of the true lumen **115** to hardly fall off from the inner wall.

The number of the elastic members **2** is set at ten, and accordingly, the pushing force of each elastic member **2** against the inner wall of the true lumen **115** is reduced while the bloodstream of the true lumen **115** is dilated definitely. This prevents the pushing force of the elastic members **2** from working greatly and locally on the inner layer at dilation of the bloodstream of the true lumen **115** to thus reduce the burden on the inner layer. Even when the number of the elastic members **2** is set at eight or nine, the same effects of reducing the burden on the inner layer at dilation of the bloodstream of the true lumen **115** can be obtained.

Setting of the length in the center line direction of the bloodstream dilating device **1** at approximately 40 mm to approximately 60 mm enables dilation of only a necessary part of the true lumen **115.** This prevents an unnecessary part of the true lumen **115** from being dilated to suppress damage on the inner layer.

The contact parts of the elastic members **2** with the inner wall are made long by setting the length in the center line direction of the bloodstream dilating device **1** longer than the outer diameter of a part thereof corresponding to the straight parts **5,** thereby stabilizing the bloodstream dilating device **1** in the true lumen **115.**

Hence, in the bloodstream dilating device 1 in accordance with Embodiment 1, the elastic members **2** push the inner wall of the true lumen **115** outward to dilate the bloodstream of the true lumen **115** and to narrow the bloodstream of the false lumen **114.** The linear elastic members **2** less inhibit the blood flow in the bloodstream of the true lumen **115.** In consequence, the amount of the blood flowing in the bloodstream of the true lumen **115** increases to secure the amount of the blood flowing to the visceral arteries **112, 113,** thereby suppressing ischemia of the viscera.

Each elastic member **2** is formed so as to extend in the blood flow direction of the true lumen **115** to less inhibit the blood flow in the bloodstream of the true lumen **115.**

Further, the one end parts of the elastic members **2** are combined with each other while the other end parts thereof are combined with each other to prevent the elastic members **2** from separating from each other in an operation for indwelling the bloodstream dilating device **1** into the bloodstream of the true lumen **115.** This facilitates the operation for indwelling the bloodstream dilating device **1** into the bloodstream of the true lumen **115.**

The distal ends of the protruding portions **6** of the elastic members **2** point toward the peripheral side of the blood flow direction to suppress falling off of the protruding portions **6** from the inner wall of the true lumen **115.** Hence, displacement of the bloodstream dilating device 1 toward the peripheral side of the bloodstream is suppressed to achieve desired dilation of the bloodstream of the true lumen **115.**

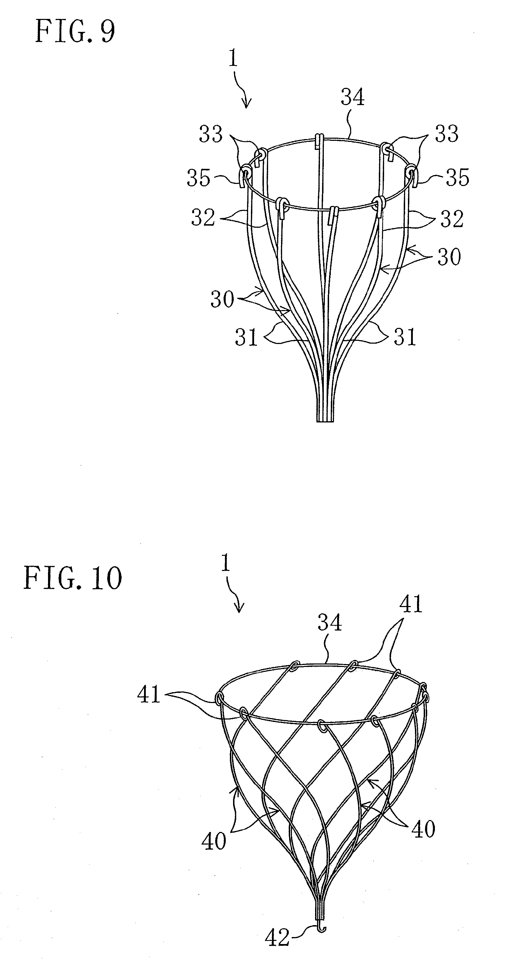

The bloodstream dilating device 1 may have a shape as in Modified Example 1 shown in FIG. **9****.** In Modified Example 1, the number of the elastic members **2** is set at eight and one end parts of the elastic members **30** are combined with each other. A curved part **31** curved radially away from the center line of the bloodstream dilating device **1** is formed on a part opposite the one end part of each elastic member **30**. At the other end parts of the elastic members **30,** straight parts **32** are formed which extend straight to the corresponding curved parts 31 in parallel with the center line of the bloodstream dilating device **1.** A hole **33** through which an annular member **34** formed of a thread or the like is inserted is formed on the other end part of each elastic member **30.** The annular member **34** may be made of a bioabsorbable material, such as polyglycolic acid fiber, collagen fiber, or the like, for example. The kind of the bioabsorbable material is not limited as long as it is one conventionally used as a material of a suture, a sutura aiding member, and the like in the medial fields. For example, it may be MEDIFIT (registered trademark) produced by JMS Co., Ltd. The annular member 34 may be made of a stretchable material, such as rubber, or the like, for example.

The annular member **34** connects the other end parts of the elastic members **30** to each other to suppress excessive outward displacement of the elastic members **30** in the radial direction of the center line. The holes **33** are formed by bending the other end parts of the elastic members **30** annually. Protruding portions **35** are provided adjacent to parts forming the holes **33** at the other end parts of the elastic members **30.** The protruding portions **35** are to be caught at the inner wall of the true lumen **115.**

Formation of the other end parts of the elastic members **30** as in Modified Example 1 allows the other end parts of the elastic members **30** to be located at the outer periphery of the bloodstream of the true lumen **115** apart from the center of the bloodstream thereof when the bloodstream dilating device **1** is indwelled in the true lumen **115.** Accordingly, the elastic members **30** further less inhibit the blood flow in the bloodstream of the true lumen **115.**

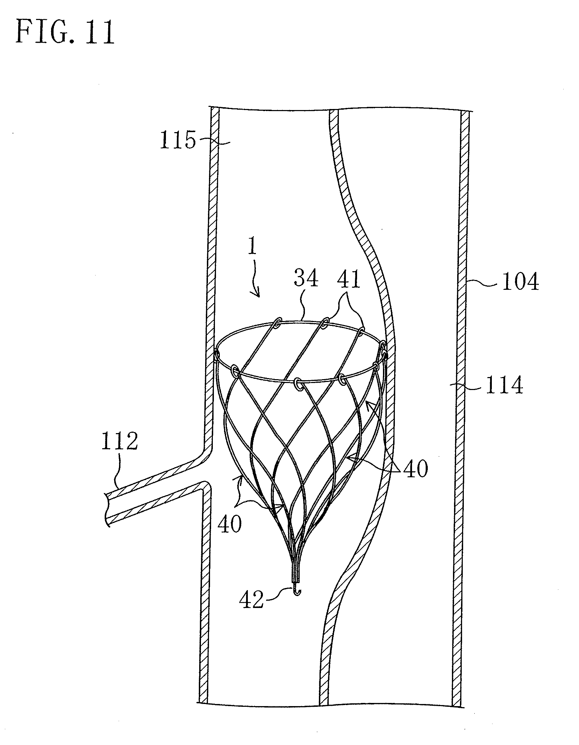

The bloodstream dilating device 1 may be composed of elastic members **40** in a spiral shape as in Modified Example 2 shown in FIG. **10** and FIG. 11. The one end parts of the elastic members **40** (the lower end parts in FIG. **10****)** are located on or near the center line of the bloodstream dilating device **1** at the one end part in the center line direction thereof and are integrated with each other. Parts (the upper parts in FIG. **10****)** opposite the one end parts of the elastic members **40** separate radially outward from the center line of the bloodstream dilating device 1 and are spiral about the center line of the bloodstream dilating device **1.** Holes **41** through which the annular member **34** in Modified Example 1 passes are formed in the other end parts of the elastic members **40.** The annular member **34** connects the other end parts of the elastic members **40** to each other to suppress excessive outward displacement in the radial direction of the center line. With the use of the bloodstream dilating device **1** of Modified Example 2, the spiral parts of the elastic members **40** dilate the bloodstream of the true lumen **115** desirably as well. The bloodstream dilating device **1** of Modified Example 2 may be provided with protruding portions as in Modified Example 1 for being caught at the inner wall of the true lumen **115.**

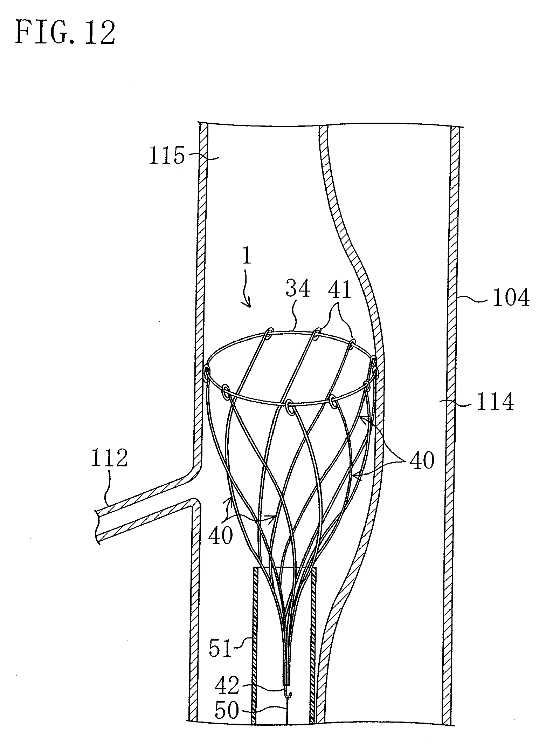

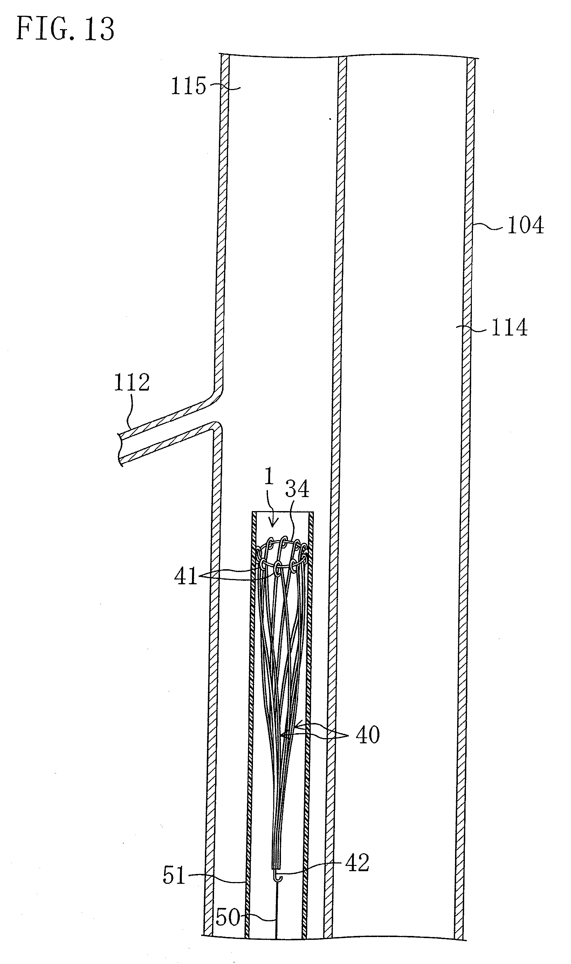

In the bloodstream dilating device **1** of Modified Example 2, a hook **42** is provided at the one end parts of the elastic members **40,** as shown in FIG. **12** and FIG. **13****,** so that the bloodstream dilating device **1** can be withdrawn from the true lumen **115** when the symptom of the aortic dissection becomes stable. The hook **42** is formed integrally with the elastic members **40.** For withdrawing the bloodstream dilating device **1,** a withdrawing tool **50** and a catheter **51** are used. The withdrawing tool **50** is formed of a wire capable of being inserted in the catheter **51** and has one end in a shape capable of being hooked by the hook **42.** The withdrawing tool **50** and the catheter **51** are inserted into the aorta **100** from an artery of a patient's lower limb. Then, the one end part of the withdrawing tool **50** is pushed out from the distal end of the catheter **51** and is then hooked by and connected to the hook **42.** Then, the withdrawing tool **50** is pulled from the base end of the catheter **51.** In so doing, since the elastic members **40** are in a spiral shape about the center line of the bloodstream dilating device **1,** the bloodstream dilating device **1** reduces its diameter smoothly by puling the catheter **51** and rotating the hook **42** in the spiral direction of the elastic members **40** by the withdrawing tool **50.** After being accommodated in the catheter **51** in this way, the bloodstream dilating device **1** is taken out from the patient's body together with the catheter **51.** The hook **42** may be provided for any bloodstream dilating devices other than that in Modified Example 2. Provision of the hook **42** facilitates withdrawal of the bloodstream dilating device **1.**

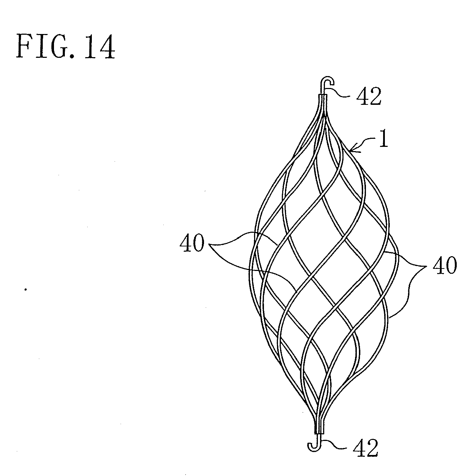

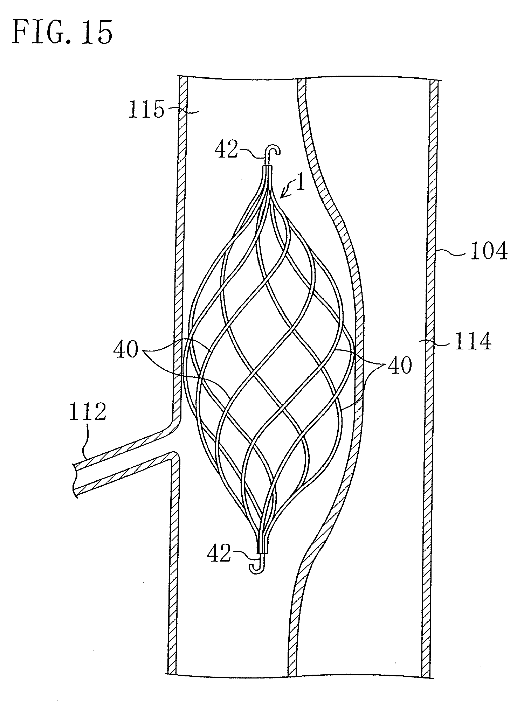

Furthermore, the bloodstream dilating device **1** may be in a substantially lemon shape by braiding the spiral elastic members **40,** as in Modified Example 3 shown in FIG. **14** and FIG. **15****.** The elastic members **40** in a linear shape are made of a super-elastic material, such as a Ni-Ti alloy or the like and have a line diameter of approximately 0.2 mm. The one ends (the lower ends in FIG. **14****)** of the elastic members **40** are located on or near the center line of the bloodstream dilating device **1** at the one end in the center line thereof and are combined and integrated with each other. Parts (the upper parts in FIG. **14****)** opposite the one end parts of the elastic members **40** separate radially outward from the center line of the bloodstream dilating device **1** and are formed spirally about the center line of the bloodstream dilating device **1.** The other end parts of the elastic members **40** are arranged just the same as the one end parts thereof to be combined and integrated with each other. The same hook **42** as that in Modified Example 2 is provided at each end in the center line direction of the bloodstream dilating device **1.** Accordingly, the bloodstream dilating device **1** of Modified Example 3 can be withdrawn with the use of a withdrawing tool. The elastic members **40** are subjected to a twisting treatment. The twisting treatment is a treatment of applying twisting force to an elastic member **40** by rotating one end part of the elastic member **40** about the center line thereof with the other end part thereof fixed. The twisting treatment on each elastic member **40** makes the elastic members **40** to have spirally waved surfaces, so that the elastic members 40 hardly slip on the inner layer of the true lumen **115** when the elastic members **40** are in contact therewith. Hence, displacement of the bloodstream dilating device **1** is suppressed.

### (Embodiment 2)

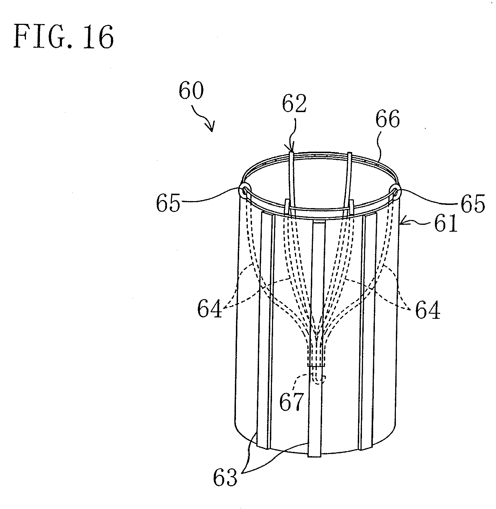

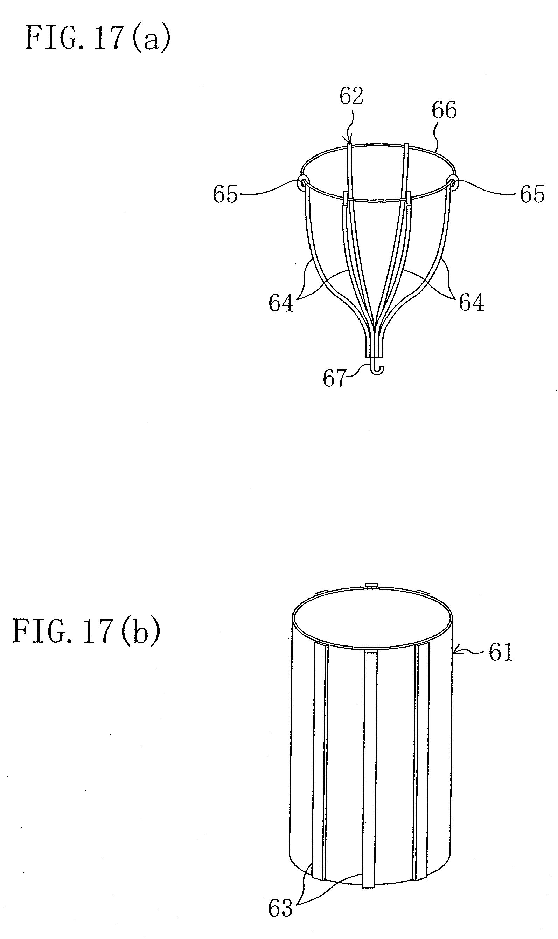

FIG. **16** shows an aortic dissection treating device **60** in accordance with Embodiment 2 of the present invention. The treating device **60** is used for treating aortic dissection classified as DeBakey IIIa type or DeBakey IIIb type and includes, as shown in FIG. **17****,** an obturator **61** for occluding an entry port **E** formed in the inner layer of an aorta **100** and a fixing member **62** for fixing the obturator **61** to the inner layer of the aorta **100.** The obturator **61** made of a flexible, elastic, and thin film-shaped resin material and is formed in a cylindrical shape. The obturator **61** is readily deformed by force to such an extent of the pressure of the blood flowing in the aorta **100.** The obturator **61** has a diameter in the range between 30 mm and 40 mm, both inclusive and a length in the center line direction in the range between 30 mm and 60 mm, both inclusive.

At the outer face of the obturator **61,** striped protrusions **63** are integrally formed which protrude radially outward of the obturator **61** and extend in the center line direction. The thickness of the parts at which the striped protrusions **63** are formed are greater than the other parts of the obturator **61.** The plurality of striped protrusions **63** are formed at regular intervals around the periphery of the obturator **61.** The striped protrusions **63** suppresses turning up of the obturator **61** by the pulsation of the blood flowing in the aorta **100.** The striped protrusions **63** serve as a reinforcing part in the present invention.

The fixing member **62** includes a plurality of linear elastic members **64,** as shown in FIG. 17(a). Within the obturator **61,** the elastic members **64** extend in the center line direction of the obturator **61** so as to form a cylindrical shape having a center line agreeing with the center line of the obturator **61,** as shown in FIG. **16****.** The elastic members **64** are made of a metal material, a resin material, or the like having a spring characteristic, such as a Ni-Ti alloy, a stainless steel, or the like, for example, and are formed so as to push the inner layer of the aorta **100** radially outward when it is inserted in the true lumen **115.**

One end parts of the elastic members **64** (the lower end parts in FIG. **16** and FIG. **17****)** are combined and integrated with each other on the center line of the fixing member **62** at one end part in the center line direction thereof. The parts (the upper parts in FIG. **16** and FIG. **17****)** opposite the one end parts of the elastic members **64** are curved radially away from the center line of the fixing member **62** so that spaces are formed between the peripherally adjacent elastic members **64** other than the one end parts thereof. The other end parts of the elastic members **64** are located on one plane substantially intersected at a right angle with the center line of the fixing member **62.** The distances between the other end parts of the elastic members **64** and the center line of the fixing member **62** are set substantially equal to each other.

The outer diameter of the fixing member **62** increases as it goes toward the other end parts in the center line thereof (upward in FIG. **16** and FIG. **17****)** by the curved parts of the elastic members **64.** The outer diameter at the other end parts in the center line direction of the fixing member **62** is set substantially equal to the inner diameter of the obturator **61.** The length in the center line direction of the fixing member **62** is set shorter than the length in the center line direction of the obturator **61.**

Holes **65** are formed at the other end parts of the elastic members **64.** The holes **65** are formed by bending the corresponding other end parts of the elastic members **64** annually and outward of the fixing member **62** so as to be formed in the elastic member **64.** An annular member **66** formed of a thread or the like is inserted in each hole **65.** The annular member **66** connects the other end parts of the elastic members **64** to each other in the peripheral direction. This suppresses excessive displacement of the other end parts of the elastic members **64** in the direction that the diameter of the fixing member **62** increases. The annular member **66** and the elastic members **64** compose the fixing member **62.**

A hook **67** protruding in the center line direction of the fixing member **62** from the fixing member **62** is provided at the one end part in the center line direction of the fixing member **62.** The hook **67** is integrally formed with the elastic members **64** and is curved so as not to allow a withdrawing tool **68** hooked, which will be described later, to fall off therefrom. The hook **67** serves as an engaging part in the present invention.

As shown in FIG. **16****,** the other end part in the center line direction of the fixing member **62** protrudes from an end opening of the obturator **61.** In this state, the other end part in the center line direction of the fixing member **62,** namely, a part thereof which pushes the inner layer of the aorta **100** radially outward is allowed to adhere to the inner face of the obturator **61** by means of an adhesive or the like, for example. Alternatively, the obturator **61** may be fixed to the fixing member **62** by means of a tread or the like, for example.

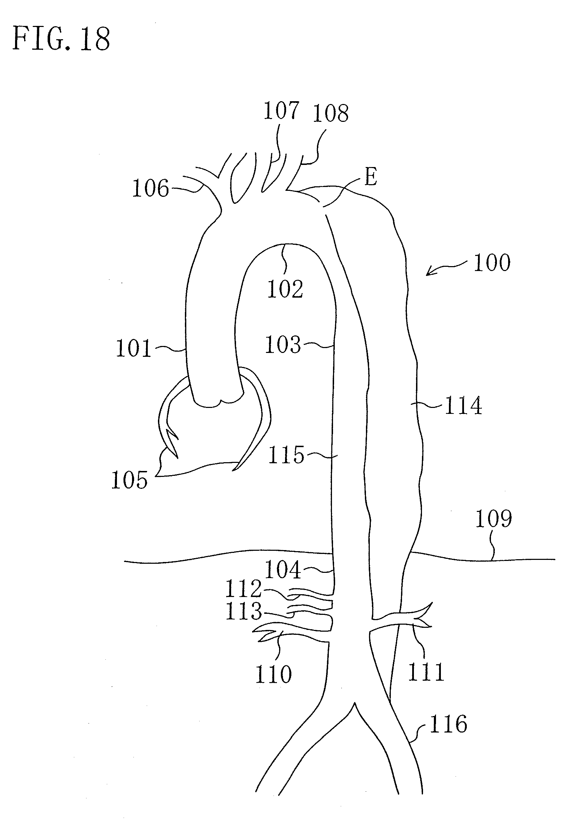

Description will be given next on a case for treating aortic dissection with the use of the thus constructed aortic dissection treating device **60.** First, an aorta **100** and a pathologic condition of the aortic dissection will be described with reference to FIG. **18****.** The aorta **100** includes an ascending aorta **101,** an arcuate aorta **102,** a thoracic descending aorta 103, and a ventral aorta **104.** Coronary arteries **105** branch from the starting part of the ascending aorta **101.** A right brachiocephalic artery **106,** a left common carotid artery **107,** and a left subclavian artery **108** branch from the arcuate aorta **102.** Four main ventral branches of a left renal artery **110,** a right renal artery **111,** a celiac artery **112,** and a superior mesenteric artery **113** branch from the ventral aorta **104.** Reference numeral **109** denotes a diaphragm.

When an entry port **E** is formed in the inner layer of the arcuate aorta **102** on the peripheral side of the branch part of the left subclavian artery **108,** the blood in the arcuate aorta **102** flows between the inner layer and the outer layer through the entry port **E.** The blood flowing between the inner layer and the outer layer breaks the inner layer to peel the inner layer from the outer layer toward the peripheral side, thereby forming a false lumen **114.** In other words, the aortic dissection forms the false lumen **114** beside the true lumen **115** forming a normal bloodstream in the arcuate aorta **102,** the thoracic descending aorta **103,** and the ventral aorta **104.** The inner diameter of the true lumen **115** is smaller than that of the normal arcuate aorta **104** and that of the false lumen **114.** The entry port **E** is in a slit shape long in the peripheral direction of the arcuate aorta **102** and has a dimension in the longitudinal direction of approximately 20 mm to approximately 30 mm and a dimension in the widthwise direction of approximately 10 mm.

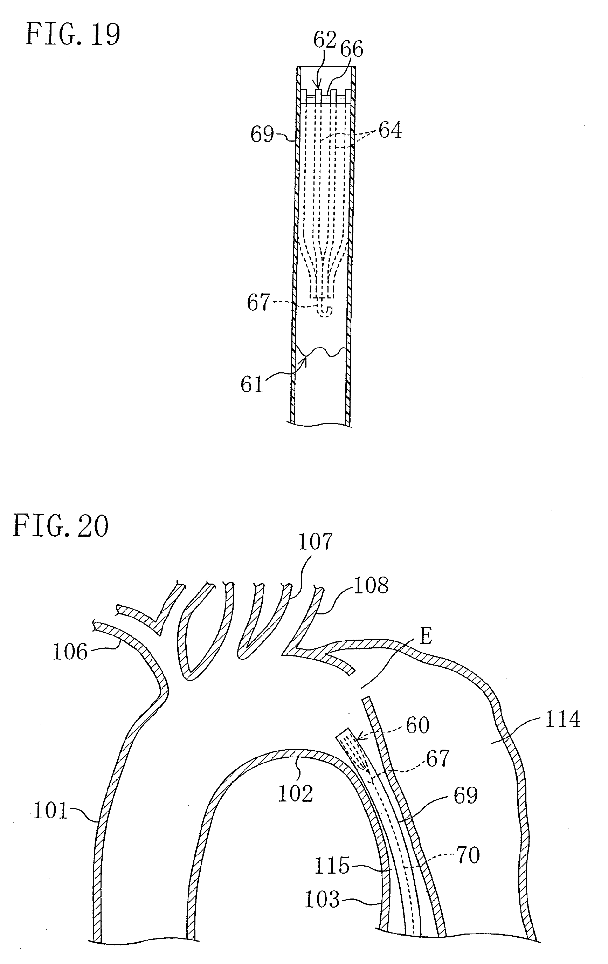

The treating device **60** accommodated in a catheter **69** is inserted up to the arcuate aorta **102,** as shown in FIG. **19** and FIG. **20****.** The catheter **69** is one used in the medical fields and is made of a flexible resin material. For inserting the treating device **60** into the catheter **69,** the fixing member **62** is reduced in its outer diameter more than the inner diameter of the catheter **69** by elastically deforming the elastic members **64** radially inward of the fixing member **62.** Reduction in the outer diameter of the fixing member **62** produces slack in the obturator **61.** The slacking part of the obturator **61** is folded in the peripheral direction. In this way, the treating device **60** set in the size capable of being inserted in the catheter **69** is inserted from the one end in the center line direction of the fixing member **62** into the distal end in the insertion direction of the catheter **69** so as to be accommodated in the catheter **69.** A wire **70** has been inserted in the catheter **69** to be engaged at one end thereof with the hook **67** of the fixing member **62.** The other end of the wire **70** is protruded from the base end of the catheter **69.**

A skin of a patient's lower limb (not shown) is incised, and the catheter **69** is inserted into an artery of the lower limb. The catheter **20** is moved in the artery in the insertion direction to insert the distal end part thereof from the common iliac artery **116** shown in FIG. **18** through the ventral aorta **104** and the thoracic descending aorta **103** and is then positioned at a predetermined point on the peripheral side of the entry port **E** in the arcuate aorta **102,** as shown in FIG. **20****.**

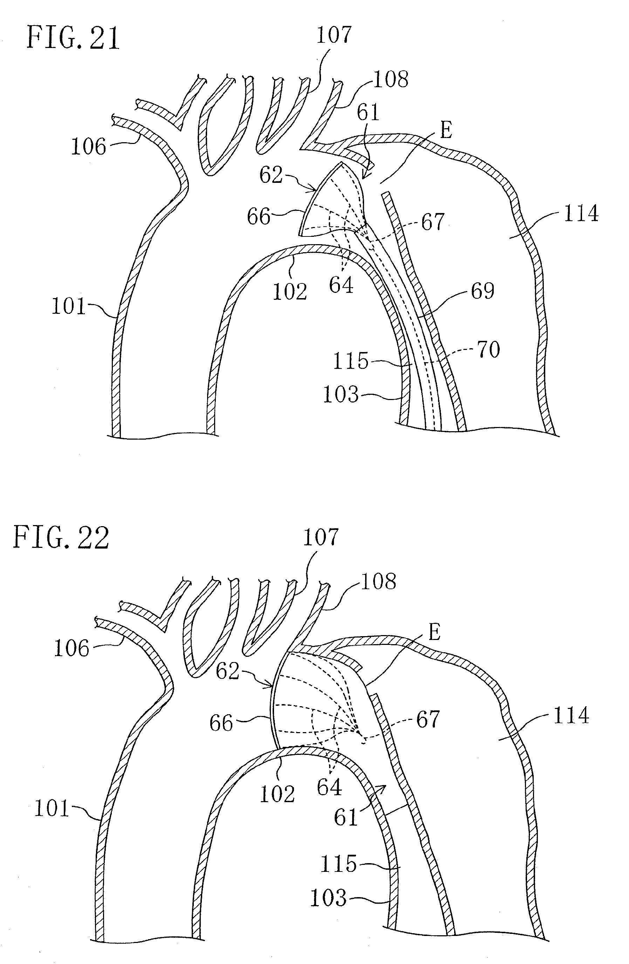

Thereafter, the other end part of the wire **70** is pushed to the catheter **69** from the base end of the catheter **69** to push out the treating device **60** from the distal end the catheter **69** into the true lumen **115,** as shown in FIG. **21****.** The predetermined point where the distal end part of the catheter **69** is to be positioned is set so that the other end part in the center line direction of the fixing member **62** of the treating device **60** pushed out from the catheter **69** is located on the central side of the entry port **E.** Specifically, the other end part in the center line direction of the fixing member **62** is set at a point approximately 10 mm apart on the central side from the entry port **E.**

As the treating device **60** is pushed out from the catheter **69,** the other end parts in the center line direction of the elastic members **64** of the fixing member **64** are deformed radially outward to increase the outer diameter of the fixing member **62.** The increase in the outer diameter of the fixing member **62** reduces the slack in the peripheral direction of the obturator **61.** When the treating device **60** is pushed out from the catheter **69** completely, the other end parts in the center line direction of the elastic members **64** of the fixing member **62** are deformed radially outward of the arcuate aorta **102** to push the inner layer thereof, as shown in FIG. **22****.**

Accordingly, the obturator **61** is held between the elastic members **64** and the inner layer to cover the entry port **E.** The striped protrusions **63** formed around the obturator **61** suppress turning up of the obturator **61** into the false lumen **114** through the entry port **E** which is caused by the flow of the blood flowing into the false lumen **114** from the true lumen **115** through the entry port **E.** The cylindrical shape of the obturator **61** enables occlusion of the entry port **E** irrespective of the position in the peripheral direction of the obturator **61.**

The blood flowing from the central side of the arcuate aorta **102** flows into the obturator **61,** passes through the spaces between the elastic members **64,** and flows then to the peripheral side of the true lumen **115.** The blood flow direction substantially agrees with the direction that the elastic members **64** extend. As well, the direction that the striped protrusions **63** extend substantially agrees with the blood flow direction.

The pressure of the blood flowing in the obturator **61** produces radially outward force working on the inner face of the obturator **61.** The obturator **61** receiving the blood pressure, which is in a thin film shape, is readily deformed along and is in contact with the periphery of the entry port **E** of the inner layer.

The obturator **61** is deformed by the blood pressure to be in contact with the periphery of the entry port **E** in this way, and therefore, the entry port **E** can be occluded without necessitating precise design of the obturator **61** for each patient's true lumen **115.** Thus, the flow of the blood flowing into the false lumen **114** through the entry port **E** is blocked. The blocking of the flow of the blood flowing into the false lumen **114** through the entry port **E** allows less or no blood to flow into the false lumen **114,** so that the blood in the false lumen **115** forms a thrombus in an earlier phase to stabilize the pathologic condition of the aortic dissection. During the treatment, the patient's blood pressure is lowered by a medicine or the like. The thrombus will gradually grow small and disappears in due course.

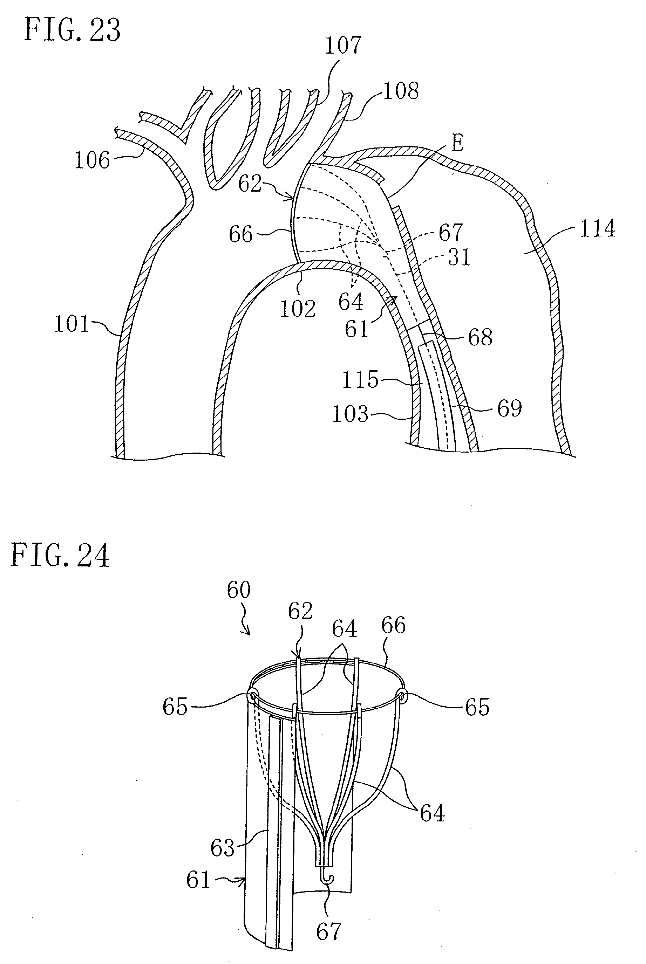

When the pathologic condition of the aortic dissection becomes stable and the treating device **60** becomes unnecessary, the treating device **60** is withdrawn from the true lumen **115.** For the withdrawal, a catheter **69** and the withdrawing tool **68** are used, as shown in FIG. **23****.** The withdrawing tool **68** is formed of a wire capable of being inserted in the catheter **69** and has at one end thereof a joint **71** in a shape to be hooked by the hook **67** of the fixing member **62.** The catheter **69** and the withdrawing tool **68** are inserted from an artery of the patient's lower limb to the arcuate aorta **102** in the similar manner to an operation for inserting the treating device **60.** Then, the joint **71** of the withdrawing tool **68** is protruded from the distal end of the catheter **69** to be joined by being hooked by the hook **67,** and then, the withdrawing tool **68** is pulled from the base end of the catheter **69,** so that the fixing member **62** is accommodated into the catheter **69** from the one end in the center line direction of the fixing member **62.** In the process of accommodating the fixing member **62** into the catheter **69,** the elastic members **64** are elastically deformed radially inward of the fixing member **62** to reduce the outer diameter of the fixing member **62.** On the other hand, the obturator **61** is in contact with and slides on the catheter **69** in the process of being accommodated into the catheter **69,** with a result that the obturator **61** is accommodated with a part thereof other than a part adhering to the fixing member **62** reversed. After being accommodated into the catheter **69** in this way, the treating device **60** is taken out from the patient's body together with the catheter **69.**

Hence, according to the aortic dissection treating device **60** of Embodiment 2, the film-shaped obturator **61** is inserted into the true lumen **115,** is fixed to the inner layer of the arcuate aorta **102** by means of the fixing member **62,** and is then allowed to be in contact with the periphery of the entry port **E** of the inner layer by the pressure of the blood in the true lumen **115.** Accordingly, the entry port **E** can be occluded without necessitating precise design of the obturator **61** for each patient. This eliminates the need to prepare a device, equipment, and the like for precisely designing the obturator **61,** with a result that the hypotensive therapy can be carried out in various medical sites.

With the plurality of elastic members **64** of the fixing member **62,** plural parts of the obturator **61** can be held between the elastic members **64** and the inner layer. This suppresses displacement of the obturator **61** to occlude the entry port **E** definitely.

With the hook **67** at the fixing member **62,** the withdrawing tool **68** can be allowed to be engaged with the hook **67,** thereby attaining unfailing and easy withdrawal of the aortic dissection treating device **60** from the inside of the true lumen **115.**

Further, radially extending and cylindrical obturator **61** can occlude the entry port **E** without necessitating rotation of itself in the radial direction of the true lumen **115** within the true lumen **115,** thereby facilitating a treatment for occluding the entry port **E** by the obturator **61.**

The striped protrusions **63** formed around the obturator **61** suppress turning up of the obturator **61** into the entry port **E** to occlude the entry port **E** definitely.

In addition, the elastic members **64** extend in the blood flow direction when the fixing member **62** is set in the true lumen **115,** so that the blood flow in the true lumen **115** is less inhibited by the elastic members **64,** thereby securing the amount of the blood flowing in the true lumen **115.**

Though the obturator **61** is in a cylindrical shape in Embodiment 2, the shape thereof may be cut in the peripheral direction of the obturator **61,** as in Modified Example 1 shown in FIG. **24****.** The obturator **61** in Modified Example 1 is substantially in a rectangular shape that covers a half part in the peripheral direction of the fixing member **62.**

The treating device **60** of Modified Example 1 is suitable for the case, for example, where the entry port **E** is formed near a branching artery, such as the left subclavian artery **108** or the like. In this case, when the cut part of the obturator **61** is allowed to agree with the inlet of the branching artery in inserting the treating device **60** into the true lumen **115,** the inlet of the branching artery can be prevented from being occluded by the obturator **61** while the entry port **E** can be occluded, thereby maintaining the blood flow to the branching artery.

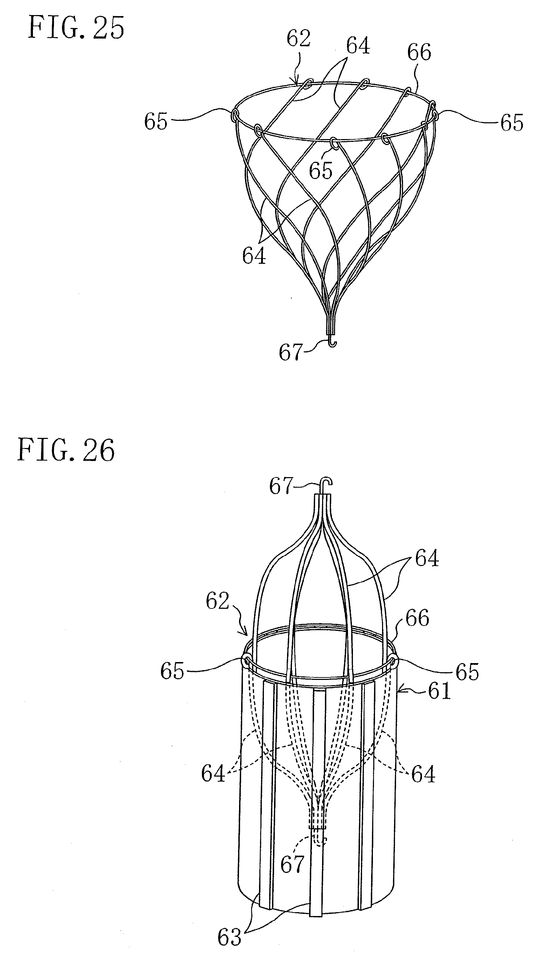

The fixing member **62** may have a construction as in Modified Example 2 shown in FIG. **25****,** for example. In Modified Example 2, the elastic members **64** are in a thin linear shape and the one end parts thereof are combined and integrated with each other at the one end in the center line direction of the fixing member **62.** The parts opposite the one end parts of the elastic members **64** separate radially away from the center line of the fixing member **62** and are curved peripherally in a spiral shape. Holes **65** are formed at the other end parts of the elastic members **34.** As in Modified Example 2, the thin linear elastic members **64** less inhibit the blood flow in the true lumen **115** when the fixing member **62** is inserted in the true lumen **115.** Further, in Modified Example 2, the elastic members **64** are in a spiral shape about the center line of the fixing member **62.** Accordingly, when the hook **67** is pulled into the catheter **69** while being rotated in the spiral direction of the elastic members **64** by the withdrawing tool **68** after the withdrawing tool **68** is hooked by the hook **67** for withdrawal by the withdrawing tool **68,** the fixing member **62** reduces its diameter smoothly to be accommodated in the catheter **69.**

The shape of the obturator **61** is not limited to the cylindrical and rectangular shapes, but may be any shape as long as it can occlude the entry port **E.** Further, the striped protrusions **63** of the obturator **61** are provided around the outer face of the obturator **61** in Modified Example 2, but may be provided around the inner face of the obturator **61,** namely, on the fixing member **62** side. Alternatively, the striped protrusions **63** of the obturator **61** may be dispensed with.

As in Modified Example 3 shown in FIG. **26****,** the other end parts in the central line direction of the elastic members **64** may be protruded from the obturator **61** so that the protruded parts also push the inner layer of the true lumen **115.** The other end parts in the center line direction of the elastic members **64** in Modified Example 3 have substantially the same shape as the one end parts of the elastic members **64** which are covered with the obturator **61.** In other words, the other end parts in the central line direction of the elastic members **64** are combined and integrated with each other on the center line of the fixing member **62** at the other end part in the center line direction thereof. A hook **67** similarly to that at the one end part of the fixing member **62** is provided at the other end part in the center line direction thereof. In Modified Example 3, the hook **67** is provided at each end in the center line direction of the fixing member **62,** so that the treatment device **60** can be withdrawn by the withdrawing tool **68** from the true rumen **115** from either of the peripheral side or the central side of the aorta **100.**

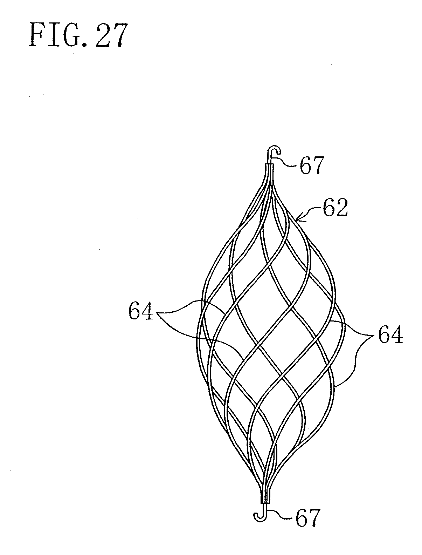

The fixing member **62** may have a construction as in Modified Example 4 shown in FIG. **27****.** In Modified Example 4, The elastic members **64** may be made of a super-elastic material of a Ni-Ti alloy or the like, for example, and have a line diameter of approximately 0.2 mm. The number of the elastic members **64** is set at eight, and the one end parts of the elastic members **64** are combined and integrated with each other at the one end in the center line direction of the fixing member **62.** The parts opposite the one end parts of the elastic members **64** separate radially away from the center line of the fixing member **62** and are curved peripherally in a spiral shape. The other end parts of the elastic members **64** are combined and integrated with each other at the other end in the center line direction of the fixing member **62.** A hook **67** is provided at each end in the center line direction of the fixing member **62.** Though not shown, the obturator 61 is fixed to the elastic members **64** by means of an adhesive, a bioabsorbable thread, or the like. Each elastic member **64** is subjected to a twisting treatment. The twisting treatment is a treatment of applying twisting force to an elastic member **64** by rotating one end part of the elastic member 64 about the center line thereof with the other end part thereof fixed. The twisting treatment on the elastic members **46** makes the elastic members 64 to have spirally waved surfaces so that the elastic members **64** hardly slip on the inner layer of the true lumen **115** when the elastic members **64** are in contact therewith.

The fixing member **62** in Modified Example 4 is made of a super-elastic material. Therefore, when the hooks **67** are pulled so as to separate from each other in the center line direction of the fixing member **62,** the fixing member **62** reduces in its diameter entirely and elongates in the center line direction thereof. When the pulling force is released, the fixing member **62** recovers to its original shape. This enables easy insertion of the fixing member **62** into the catheter **69** at the beginning of the treatment. Further, when one of the hooks **67** is pulled by the withdrawing tool **68** for withdrawal from the true lumen **115,** the fixing member **62** can be accommodated into the catheter **69** easily.

The obturator **61** may be made of a bioabsorbable material, such as polyglycolic acid fiber, collagen fiber, or the like, for example. The kind of the bioabsorbable material is not limited specifically as long as it is a material conventionally used as a material of a suture, a sutura aiding member, and the like in the medial fields. For example, it may be MEDIFIT (registered trademark) produced by JMS Co., Ltd. When the blood in the false lumen **114** is coagulated to form a thrombus, the obturator **61** made of the bioabsorbable material is integrated with the thus formed thrombus to cling to the inner layer. Then, the obturator **61** is gradually absorbed into the inner layer of the aorta **100** as time passes and disappears from the aorta **100** in due course. Accordingly, only withdrawal of the fixing member **62** by the withdrawing tool **68** is necessary. In other words, it is unnecessary to take out the obturator **61** from the aorta **100.** This attains low invasion treatment.

The annular member **66** may be made of the aforementioned bioabsorbable material or rubber or the like, for example. The annular member **66** made of a bioabsorbable material will disappear from the aorta **100** in due course. Accordingly, only the elastic members 64 are required to be withdrawn by the withdrawing tool **68** after the pathologic condition of the dissection becomes stable. This attains low invasion treatment as well.

### Industrial Applicability

As described above, the bloodstream dilating device in accordance with the present invention is suitable for dilating a bloodstream of a true lumen of an aorta where aortic dissection occurs, and the aortic dissection treating device is suitable for treating DeBakey IIIa type or DeBakey IIIb type aortic dissection where an entry port is formed in a part somewhat on the peripheral side of the left subclavian artery of the arcuate aorta, for example.

## Claims

1. A bloodstream dilating device to be indwelled in a bloodstream of a true lumen of an aorta where aortic dissection, which forms a false lumen in the aorta beside the true lumen by blood in the aorta flowing between an inner layer and an outer layer of the aorta, occurs, the bloodstream dilating device comprising:
an elastic member for pushing an inner wall of the true lumen outward of the true lumen.

2. The bloodstream dilating device of claim 1,
wherein the elastic member includes a plurality of elastic members, and
the elastic members are formed so as to extend linearly in a direction of a blood flow in the true lumen and are spaced in a peripheral direction of the true lumen.

3. The bloodstream dilating device of claim 2,
wherein one end parts of the elastic members are combined with each other while the other end parts thereof are combined with each other, and intermediate parts of the elastic members are formed so as to push the inner wall of the true lumen.

4. The bloodstream dilating device of claim 2,
wherein one end parts of the elastic members are combined with each other while the other end parts thereof are formed so as to push the inner wall of the true lumen.

5. The bloodstream dilating device of claim 1,
wherein the elastic member is provided with a protruding portion formed so as to be caught at the inner wall of the true lumen.

6. The bloodstream dilating device of claim 5,
wherein the protruding portion points toward the peripheral side in a direction of a blood flow in the true lumen.

7. An aortic dissection treating device for treating aortic dissection occurring due to flow of blood in an aorta flowing between an inner layer and an outer layer of the aorta through an entry port formed in the inner layer of the aorta, the aorta dissection treating device comprising:
an obturator in a film shape larger than the entry port for occluding the entry port by being inserted into the true lumen of the aorta and being in contact with a periphery of the entry port in the inner layer by pressure of the blood flowing in the aorta; and
a fixing member for fixing the obturator to the inner layer.

8. The aortic dissection treating device of claim 7,
wherein the fixing member is composed of an elastic member formed so as to be elastically deformed radially outward of the aorta to push the inner layer, and
the obturator is mounted on a part of the elastic member which pushes the inner layer.

9. The aortic dissection treating device of claim 8,
wherein the elastic member is formed so as to extend in a direction of the flow of the blood in the aorta.

10. The aortic dissection treating device of claim 9,
wherein the elastic member includes a plurality of elastic members spaced in a peripheral direction of the true lumen.

11. The aortic dissection treating device of claim 7,
wherein the elastic member is provided with an engaging part to be engaged with a withdrawing tool.

12. The aortic dissection treating device of claim 7,
wherein the obturator is in a cylindrical shape.

13. The aortic dissection treating device of claim 7,
wherein the obturator is cut across a peripheral direction of the true lumen.

14. The aortic dissection treating device of claim 7,
wherein the obturator is provided with a reinforcing part extending in a direction of the flow of the blood.

15. The aortic dissection treating device of claim 7,
wherein the obturator is made of a bioabsorbable material.
